# EUROPÄISCHE PATENTSCHRIFT

(11) **EP 0 733 617 B1**
(45) Veröffentlichungstag und Bekanntmachung des Hinweises auf die Patenterteilung: **02.06.1999**
(21) Anmeldenummer: 96104229.8
(22) Anmeldetag: 16.03.1996
(51) Int. Cl.: C07C 67/08, C07C 69/54

(54) **Verfahren zur kontinuierlichen Herstellung von Alkylestern der (Meth)acrylsäure**
Process for the continuous production of alkyl esters of (meth-)acrylic acid
Procédé de fabrication continue d'esters alkyliques de l'acide (méth-)acrylique

(30) Priorität: 24.03.1995 DE 19510891
(43) Veröffentlichungstag der Anmeldung: 25.09.1996
(73) Patentinhaber: BASF Aktiengesellschaft, 67063 Ludwigshafen (DE)
(72) Erfinder: Dockner, Toni, Dr., 67149 Meckenheim (DE); Exner, Herbert, Dr., 67165 Waldsee (DE); Baur, Karl Gerhard, Dr., 67063 Ludwigshafen (DE); Potthoff, Christiane, 44141 Dortmund (DE)

(56) Entgegenhaltungen:
- DE-A- 2 552 987
- GB-A- 1 173 118

## Beschreibung

Vorliegende Erfindung betrifft ein Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase im Molverhältnis von 1(Alkanol): 1 ((Meth)acrylsäure) bis 2 (Alkanol):1 ((Meth)acrylsäure) bei erhöhter Temperatur und in Gegenwart von Schwefelsäure oder einer organischen Sulfonsäure als Katalysator, bei dem man die (Meth)acrylsäure, das Alkanol und den Säurekatalysator kontinuierlich einer Reaktionszone zuführt, nach einer Verweilzeit von einigen Stunden den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten, einen Kopf druck von 0,1 bis 1 atm aufweisenden, Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und gegebenenfalls Wasser in die Reaktionszone zurückführt, aus der überschüssigen, den (Meth)acrylsäurealkylester enthaltenden organischen Phase den (Meth)acrylsäurealkylester in an sich bekannter Weise abtrennt und einen Teil des Reaktionsgemisches aus der Reaktionszone kontinuierlich austrägt.

(Meth)acrylsäure steht hier für Acryl-oder Methacrylsäure. Alkylester der (Meth)acrylsäure sind allgemein bekannt und z.B. als Ausgangsmonomere zur Herstellung wäßriger Polymerisatdispersionen von Bedeutung, die z.B. als Klebstoffe Verwendung finden.

Verfahren zur Herstellung von (Meth)acrylsäurealkylestern durch Umsetzung von (Meth)acrylsäure mit 1 bis 8 C-Atome aufweisenden einwertigen Alkanolen in homogener flüssiger Phase, bei erhöhter Temperatur und in Gegenwart protonenliefernder Katalysatoren sind bekannt und z. B. in den DE-A 1468932, 2226829, 2252334 beschrieben. Es handelt sich dabei um typische Gleichgewichtsreaktionen, bei denen der Umsetzungsgrad der (Meth)acrylsäure und des jeweiligen Alkanols zum entsprechenden Ester durch die Gleichgewichtskonstante signifikant begrenzt ist. Dies hat zur Folge, daß für eine wirtschaftliche Verfahrensführung die nicht umgesetzten Ausgangsstoffe vom gebildeten Ester abgetrennt und in die Reaktionszone zurückgeführt werden müssen. Als besonders schwierig erweist sich dabei in der Regel die Abtrennung des gebildeten Esters von nicht umgesetzter (Meth)acrylsäure, da deren Siedepunkte meist vergleichsweise eng zusammenliegen. Es wurden daher bereits verschiedene Maßnamen zur Erhöhung des Umsatzes der (Meth)acrylsäure zu den entsprechenden Estern vorgeschlagen, wie z.B. die Anwendung eines erhöhten molaren Überschußes an Alkanol gegenüber der (Meth)acrylsäure, die Entfernung des Reaktionswassers mittels eines ein geeignetes Azeotrop bildenden organischen Schleppmittels oder die Extraktion des gebildeten Esters mit einem geeigneten Lösungsmittel während der Reaktion. Diese Verfahren sind jedoch mit dem Nachteil behaftet, daß ein großer Überschuß an Alkanol zurückgenommen bzw. das Schleppmittel oder das Extraktionsmittel isoliert werden müssen. Ein erhöhter Alkanolüberschuß verstärkt zudem die Bildung dessen Dialkylether als Nebenprodukt.

Inzwischen ist bekannt, die Siedepunktsdifferenz zwischen nicht umgesetzter (Meth)acrylsäure und gebildetem Alkylester der (Meth)acrylsäure dadurch zu erhöhen, daß man den gebildeten Alkylester der (Meth)acrylsäure in wenigstens ein niedrig siedendes wäßriges Azeotrop einbettet, das neben dem (Meth)acrylsäurealkylester und Wasser noch Ausgangsalkanol enthalten kann, und den (Meth)acrylsäurealkylester als Bestandteil wenigstens eines solchen Azeotrops der, die nicht umgesetzte (Meth)acrylsäure umfassenden, Reaktionszone kontinuierlich rektifikativ zu entziehen, und so von nicht umgesetzter (Meth)acrylsäure abzutrennen. Bei kontinuierlicher Durchführung der vorstehenden Verfahrensweise wird das als Destillat anfallende wäßrige Azeotrop in wenigstens eine den (Meth)acrylsäurealkylester enthaltende organische und in wenigstens eine Wasser enthaltende wäßrige Phase aufgetrennt. Ein Teil der den (Meth)acrylsäurealkylester enthaltenden organischen Phase wird zur Einstellung der rektifikativen Trennwirkung (rektifikatives Rücklaufverhältnis) über Kopf der aufgesetzten Rektifikationszone zurückgeführt. Aus der überschüssigen, den (Meth)acrylsäurealkylester enthaltenden, organischen Phase trennt man den (Meth)acrylsäurealkylester in an sich bekannter Weise ab. Bei der Veresterung niederer (C₁-, C₂-)Alkanole reicht normalerweise das im Verlauf der Veresterung als Nebenprodukt gebildete Reaktionswasser zur Ausbildung der Zusammensetzung des wäßrigen Azeotrops aus (geeignete wäßrige Azeotropzusammensetzungen weist z.B. Azeotropic Data-III, Advances in Chemistry Series 116, American Chemical Society, Washington, D.C. (1973) aus) und wird so als Bestandteil des wäßrigen Azeotrops gleichzeitig kontinuierlich aus dem Veresterungsgleichgewicht entfernt. Mit zunehmender Kettenlänge des Alkanols ist dies jedoch nicht mehr der Fall, weshalb in diesen Fällen, über das im Verlauf der Veresterung gebildete Reaktionswasser hinaus, zusätzliches Wasser in die Reaktionszone eingeführt werden muß. In einfachster Weise wird dies dadurch realisiert, daß man einen entsprechenden Teil der bei Auftrennung des als Destillat anfallenden wäßrigen Azeotrops erzeugten wäßrigen Phase in den Veresterungsteil zurückführt. Da Wasser bezüglich des Umsatzes bei der eigentlichen Veresterungsreaktion eher stört, erfolgt die Rückführung der wäßrigen Phase vorzugsweise über Kopf der aufgesetzten Rektifikationszone. Enthält das über Kopf der aufgesetzten Rektifikationszone rektifikativ abgetrennte wäßrige Azeotrop als zusätzlichen Bestandteil Ausgangsalkanol, wird dieser aus dem nach Auftrennung des azeotropen Destillats in eine organische und eine wäßrige Phase nach deren Teilrückführung verbleibenden Überschuß an organischer und wäßriger Phase in an sich bekannter Weise abgetrennt und in die Reaktionszone zurückgeführt. Da das Ausgangsalkanol als einer der beiden Reaktanten unmittelbar an der Veresterung teilnimmt, erfolgt diese Rückführung mit Vorzug auf direktem Weg.

Die GB-1017522 offenbart ein entsprechendes Verfahren zur Herstellung von n-Butylacrylat. Als Veresterungsbedingungen empfiehlt die GB-1017522 dabei ein molares Verhältnis von Ausgangsalkanol zu Ausgangssäure von 2,3 bis 5, eine <100°C betragende Reaktionstemperatur, sowie einen auf die Gesamtmasse der Reaktanten bezogenen Gehalt an katalytisch wirksamer Schwefel- oder organischer Sulfonsäure von >0,05 bis <5 Gew.-%. Nachteilig an dieser Verfahrensweise ist der erforderliche erhöhte Überschuß an Ausgangsalkanol, der die Bildung an unerwünschtem Diakylether fördert, sowie die unter vorgenannten Bedingungen nicht voll befriedigende Ausbeute an n-Butylacrylat bezogen auf die eingesetzte Menge an Acrylsäure.

Aus der DE-PS 2552987 ist ein Verfahren zur kontinuierlichen Herstellung von Alkylestern der Acrylsäure durch Umsetzung von Acrylsäure und 1 bis 4 C-Atome aufweisenden einwertigen Alkanolen in homogener, flüssiger, an Lösungsmittel freier Phase im Molverhältnis von 1(Alkanol): 1(Acrylsäure) bis 2(Alkanol): 1(Acrylsäure) bei erhöhter Temperatur und in Gegenwart von Schwefel- oder organischer Sulfonsäure als Katalysator bekannt, bei dem man die Acrylsäure, das Alkanol und den Säurekatalysator kontinuierlich einer Reaktionszone zuführt, nach einer Verweilzeit von einigen Stunden den gebildeten Acrylsäurealkylester als Bestandteil wenigstens eines neben dem Acrylsäurealkylester als weiteren Bestandteilen aus Wasser oder Wasser und Ausgangsalkanol bestehenden wäßrigen Azeotrops über Kopf einer der Reaktionszone aufgesetzten, einen Kopfdruck von 0,1 bis 1 atm aufweisenden, Rektifikationskolonne rektifikativ abtrennt, das anfallende Destillat I in eine den gebildeten Acrylsäureester enthaltende organische und in eine wäßrige Phase auftrennt, einen Teil der organischen Phase zum Zwecke der Erzeugung einer erhöhten Trennwirkung sowie gegebenenfalls einen Teil der wäßrigen Phase zur Aufrechterhaltung der Zusammensetzung des wäßrigen Azeotrops über Kopf der Rektifikationszone zurückführt, aus der überschüssigen organischen Phase den Alkylester in an sich bekannter Weise abtrennt und einen Teil des Reaktionsgemisches aus der Reaktionszone austrägt, von Hochsiedern destillativ befreit und das dabei anfallende Destillat II in die Reaktionszone zurückführt.

Als Reaktionstemperatur empfiehlt die DE-PS 2552987 eine Temperatur von 80 bis 130°C. In den Ausführungsbeispielen wird in allen Fällen eine Temperatur von 95°C angewendet. Als Verweilzeit empfiehlt die DE-PS 2552987 eine solche von 4 bis 10 h. In allen Ausführungsbeispielen beträgt die Verweilzeit mehr als 6 h. Als einzusetzende Menge an Säurekatalysator empfiehlt die DE-PS 2552987, bezogen auf das Reaktionsgemisch, 0,1 bis 3 Gew.-% Schwefelsäure oder 1 bis 8 Gew.-% an organischer Sulfonsäure. Als in Betracht kommende organische Sulfonsäuren benennt die DE-PS 2552987 dabei lediglich Benzolsulfonsäure und Toluolsulfonsäure. In allen Ausführungsbeispielen liegt die eingesetzte Menge an Säurekatalysator, bezogen auf das Reaktionsgemisch, bei Werten ≤2,5 Gew.-%.

Vorrangige Zielsetzung der DE-PS 2552987 ist die Vermeidung unerwünschter Etherbildung aus Ausgangsalkanol. Nachteilig an der Verfahrensweise der DE-PS 2552987 ist jedoch, daß trotz destillativer Behandlung des Austrags aus dem Reaktionsgemisch und Rückführung des dabei anfallenden Destillats in die Reaktionszone die Ausbeute an Alkylacrylat, bezogen auf eingesetzte Acrylsäure, nicht zu befriedigen vermag. Ferner vermag auch die in den Ausführungsbeispielen erforderliche Verweilzeit nicht zu befriedigen. Desgleichen gilt für die Raum-Zeit-Ausbeute.

Ein kontinuierlicher Austrag eines Teils des Reaktionsgemisches ist deshalb erforderlich, weil im Verlauf der Veresterung die beteiligten α,β-monoethylenisch ungesättigten Verbindungen auch im Beisein von vorzugsweise in an sich bekannter Menge mitverwendeten Polymerisationsinhibitoren (z.B. phenolische Verbindungen wie Hydrochinon, Hydrochinonmonomethylether, aber auch p-Benzochinon, Phenothiazin, Methylenblau, Phenylendiamine und/oder Luft) in bestimmtem Ausmaß Oligo- und Polymere (Hochsieder) bilden, deren Anreicherung in der Reaktionszone es zu verhindern gilt.

Die Aufgabe der vorliegenden.Erfindung bestand daher darin, ein Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase zur Verfügung zu stellen, das die Nachteile der Verfahren des nächstliegenden Standes der Technik nicht aufweist, d.h. insbesondere nicht mehr in notwendiger Weise einer destillativen Aufarbeitung des Austrages aus der Reaktionszone bedarf, und trotzdem bei gleichzeitig verringerter Verweilzeit der Reaktanten in der Reaktionszone eine erhöhte Ausbeute an Alkyl(meth)acrylat bezogen auf eingesetzte Acrylsäure liefert, ohne daß eine nennenswerte Mehrbildung an Dialkylether des Ausgangsalkanols eintritt. Demgemäß wurde ein Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase im Molverhältnis von 1(Alkanol): 1((Meth)acrylsäure) bis 2(Alkanol): 1(Meth)acrylsäure) bei erhöhter Temperatur und in Gegenwart von Schwefelsäure oder einer organischen Sulfonsäure als Katalysator, bei dem man die (Meth)acrylsäure, das Alkanol und den Säurekatalysator kontinuierlich einer Reaktionszone zuführt, nach einer Verweilzeit von einigen Stunden den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten, einen Kopf druck von 0,1 bis 1 atm aufweisenden, Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und gegebenenfalls Wasser in die Reaktionszone zurückführt, aus der überschüssigen, den (Meth)acrylsäurealkylester enthaltenden organischen Phase den (Meth)acrylsäurealkylester in an sich bekannter Weise abtrennt und einen Teil des Reaktionsgemisches aus der Reaktionszone kontinuierlich austrägt, gefunden, das dadurch gekennzeichnet ist, daß
- die Reaktionstemperatur 100 bis 150°C beträgt,
- die Verweilzeit 1 bis 5 h beträgt und
- das in der Reaktionszone befindliche Reaktionsgemisch, bezogen auf die Menge an Reaktionsgemisch, als Säurekatalysator 5 bis 20 Gew.-% Schwefelsäure, oder eine zu einer solchen Schwefelsäuremenge äquimolare Menge einer organischen Sulfonsäure oder eine zu einer solchen Schwefelsäurenmenge äquimolare Menge eines Gemisches aus Schwefelsäure und organischer Sulfonsäure zugesetzt enthält.

Vorzugsweise enthält das in der Reaktionszone befindliche Reaktionsgemisch, bezogen auf die Menge an Reaktionsgemisch, 5 bis 15, besonders bevorzugt 5 bis 10 Gew.-% Schwefelsäure oder eine zu einer solchen Schwefelsäuremenge äquimolare Menge einer organischen Sulfonsäure oder eine zu einer solchen Schwefelsäuremenge äquimolare Menge eines Gemisches aus Schwefelsäure und organischer Sulfonsäure zugesetzt. Mit Vorteil beträgt die Untergrenze der vorgenannten Mengenbereiche der katalytisch zugesetzt enthaltenen Säure 5,5, mit besonderem Vorteil 6 und besonders bevorzugt >8 Gew.-%. Als günstige katalytisch wirksame organische Sulfonsäuren kommen insbesondere in Betracht: Methansulfonsäure, Benzolsulfonsäure, Dodecylbenzolsulfonsäure und p-Toluolsulfonsäure.

Die Reaktionstemperatur liegt vorzugsweise bei >100 bis 140°C. Mit besonderem Vorteil beträgt sie 120 bis 130°C. Die Verweilzeit in der Reaktionszone beträgt mit Vorzug 2 bis < 4 h. Mit besonderem Vorteil beträgt die Verweilzeit 3 bis < 4 h. Mit Vorteil führt man der Reaktionszone das Ausgangsalkanol und die Ausgangssäure im molaren Verhältnis 1,3 bis 1,7 : 1 zu. Ferner beträgt der Kopfdruck der Rektifikationszone in vorteilhafter Weise 1 atm. Bevorzugte Ausgangssäure ist die Acrylsäure. Als Ausgangsalkanol eignen sich für das erfindungsgemäße Verfahren neben 2-Ethylhexanol insbesondere die 1 bis 4 C-Atome aufweisenden Alkanole. Unter letzteren kommen vor allem Methanol, Ethanol, n-Propanol, iso-Propanol, n-Butanol, iso-Butanol und sec-Butanol in Betracht. Insbesondere eignet sich das erfindungsgemäße Verfahren zur Herstellung von n-Butylacrylat, weshalb sich alle Aussagen in dieser Schrift vor allem auf die Veresterung von n-Butanol mit Acrylsäure beziehen.

Hinsichtlich der Aufarbeitung des im Rahmen des erfindungsgemäßen Verfahrens aus der Reaktionszone kontinuierlich über Kopf einer Reaktifikationszone abgetrennten, den Zielester enthaltenden, wäßrigen Azeotrops, lassen sich im wesentlichen zwei Fallgestaltungen unterscheiden. Handelt es sich um ein Heteroazeotrop, wie z.B. im Fall der erfindungsgemäßen Herstellung von n-Butyl-acrylat, scheidet sich das Azeotrop nach seiner Kondensation von selbst in eine wäßrige und in eine organische Phase. Die wäßrige Phase besteht normalerweise hauptsächlich aus Wasser und etwas Alkanol, die organische Phase besteht in der Regel im wesentlichen aus dem gebildeten Ester und Alkanol. Zur Einstellung der rektifikativen Trennwirkung führt man einen entsprechenden Teil der organischen Phase über Kopf der Rektifikationszone zurück. Zur Aufrechterhaltung der Zusammensetzung des wäßrigen Azeotrops führt man einen entsprechenden Teil der wäßrigen Phase in die Reaktionszone zurück, vorzugsweise ebenfalls über Kopf der aufgesetzten Rektifikationszone. Aus dem nicht zurückgeführten Anteil der wäßrigen Phase läßt sich enthaltenes Alkanol z.B. durch Strippen (z.B. mit Luft) abtrennen und in die Reaktionszone zurückführen. Zweckmäßigerweise erfolgt die Rückführung auf direktem Weg. Das dabei anfallende im wesentlichen reine Wasser wird ausgetragen. Aus dem nicht rückgeführten Teil der organischen Phase wird der gebildete (Meth)acrylsäurealkylester in an sich bekannter Weise, z.B. gemäß der DE-A 2552987, abgetrennt. D.h. beispielsweise führt man die überschüssige organische Phase einer nachgeschalteten ersten Rektifikationskolonne zu und trennt das Alkanol über Kopf in reiner Form oder als aus Ester und Alkanol bestehendes Azeotrop ab. Vorzugsweise führt man das so abgetrennte Alkanol (in reiner oder in azeotroper Form) in die Reaktionszone zurück. Zweckmäßigerweise erfolgt die Rückführung auf direktem Weg. Die Sumpfflüssigkeit dieser ersten Rektifikationskolonne besteht im wesentlichen aus dem gewünschten Ester sowie geringen Mengen an tiefer und höher als dieser Ester siedenden Nebenprodukten. Man bezeichnet die Sumpfflüssigkeit der ersten Rektifikationskolonne deshalb auch als Roh-(Meth)acrylsäurealkylester. Bei den tiefer siedenden Nebenprodukten handelt es sich insbesondere um den Dialkylether und den Essigsäureester des Ausgangsalkanols, da die Veresterung von (Meth)acrylsäure mit Alkanolen üblicherweise ausgehend von Roh-(Meth)acrylsäure erfolgt. Diese wird überwiegend durch katalytische Gasphasenoxidation von C₃-/C₄ - Ausgangsverbindungen wie Propen oder iso-Buten erzeugt, wobei als Nebenprodukt in untergeordneten Mengen Essigsäure entsteht (vgl. z.B. DE-A 4436243). Höher siedende Nebenprodukte sind z.B. Oligo- und Polymerisate des α,β-monoethylenisch ungesättigten Zielesters. In einer nachgeschalteten Leichtsieder-Rektifikationskolonne trennt man von dem Roh-(Meth)acrylsäurealkylester üblicherweise über Kopf die leichter siedenden Nebenprodukte ab, bevor in einer dieser nachgeschalteten Schwersieder-Rektifikationskolonne der gewünschte Rein-(Meth)acrylsäurealkylester über Kopf abgetrennt werden kann. Die die schwerer siedenden Nebenprodukte enthaltende Sumpfflüssigkeit der Schwersieder-Rektifikationskolonne wird zweckmaßigerweise in die Reaktionszone zurückgeführt. Vorzugsweise auf direktem Weg. Zur Aufrechterhaltung eines stationären Zustands wird aus selbiger kontinuierlich eine geringe Menge an Reaktionsgemisch ausgetragen, um eine dortige Anreicherung an Schwersiedern zu vermeiden. Die dabei mit ausgetragene Menge an Säurekatalysator wird in die Reaktionszone ergänzt. Handelt es sich bei dem im Rahmen des erfindungsgemäßen Verfahrens aus der Reaktionszone kontinuierlich über Kopf einer Rektifikationszone abgetrennten, den Zielester enthaltenden, wäßrigen Azeotrop um kein Heteroazeotrop, so scheidet sich dieses nach seiner Kondensation nicht von selbst in eine wäßrige und in eine organische Phase. Diese Auftrennung kann jedoch in einfacher weise z.B. dadurch erzeugt 5 werden, daß man das im Azeotrop enthaltene Alkanol mittels Wasser extrahiert und das dabei anfallende Wasser/Alkanol-Gemisch rektifikativ auftrennt. Das Alkanol wird zweckmäßigerweise in die Reaktionszone zurückgeführt, vorzugsweise auf direktem Weg. Wasser wird je nach Bedarf in die Reaktionszone zurückgeführt, vorzugsweise über Kopf der aufgesetzten Rektifikationszone. Die bei der extraktiven Abtrennung des Alkanols anfallende organische Phase enthält den Zielester. Ein Teil derselben wird deshalb über Kopf der aufgesetzten Rektifikationszone zur Einstellung der rektifikativen Trennwirkung zurückgeführt. Aus der überschüssigen organischen Phase kann der Zielester in an sich bekannter Weise (z.B. wie vorstehend beschrieben) abgetrennt werden. Dabei anfallendes Alkanol wird zweckmäßigerweise der Reaktionszone zugeführt, vorzugsweise auf direktem Weg. Üblicherweise enthält das aus der Reaktionszone abgezogene wäßrige Azeotrop, bei richtiger Einstellung der rektifikativen Trennwirkung, keine Ausgangssäure. Sollte letzteres jedoch nicht der Fall sein, läßt sich diese zusammmen mit dem Alkanol extraktiv mittels Wasser abtrennen und das Extrakt anschließend in an sich bekannter Weise rektifikativ auftrennen. Selbstverständlich erfolgen beim erfindungsgemäßen Verfahren sowohl die Veresterungsreaktion als auch die rektifikativen Trennungen und Extraktionen vorzugsweise in Gegenwart von üblichen Mengen an sich üblicher Polymerisationsinhibitoren. In der Regel werden, bezogen auf die Menge der α,β-monoethylenisch ungesättigten Monomeren, 0,01 bis 0,1 Gew.-% eines geeigneten Polymerisationsinhibitors eingesetzt. Als solche kommen z.B. in Betracht phenolische Verbindungen wie Hydrochinon, Hydrochinonmonomethylether, aber auch p-Benzochinon, Phenothiazin, Methylenblau, Phenylendiamine und/oder Luft.

Das erfindungsgemäße Verfahren zeichnet sich im Vergleich zu den Verfahren des Stands der Technik durch reduzierte Verweilzeiten, eine erhöhte Ausbeute an gewünschtem Ester bezogen auf eingesetzte Ausgangssäure, einen reduzierten Austrag aus der Reaktionszone, sowie dadurch aus, daß es nicht mehr in notwendiger Weise einer Aufarbeitung des kontinuierlichen Austrags aus der Reaktionszone bedarf. Sowohl letzteres als auch die erhöhte Ausbeute sind vermutlich darauf zurückzuführen, daß der sich in der Reaktionszone einstellende stationäre Zustand kinetisch kontrolliert ist. Dabei spielt wahrscheinlich die Rückspaltung von relativ hochsiedenden Oxiestern (z.B. Alkoxypropionsäureester oder Acyloxipropionsäureester) eine zentrale Rolle. Diese Verbindungen werden z.B. dadurch gebildet, daß sich nicht umgesetzte Ausgangssäure oder Ausgangsalkanol via Michael-Addition an bereits gebildeten (Meth)acrylsäurealkylester addiert: 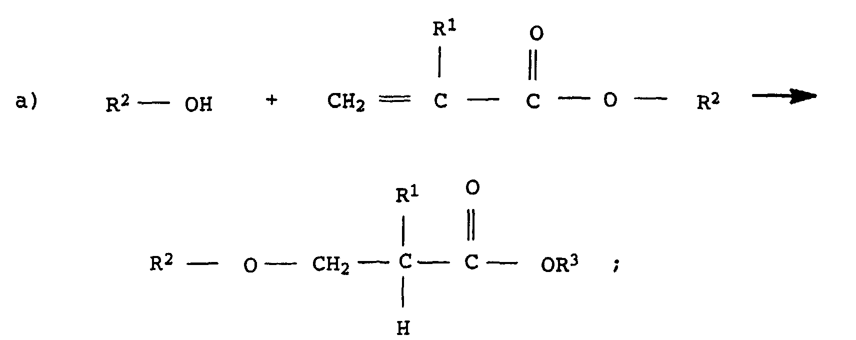 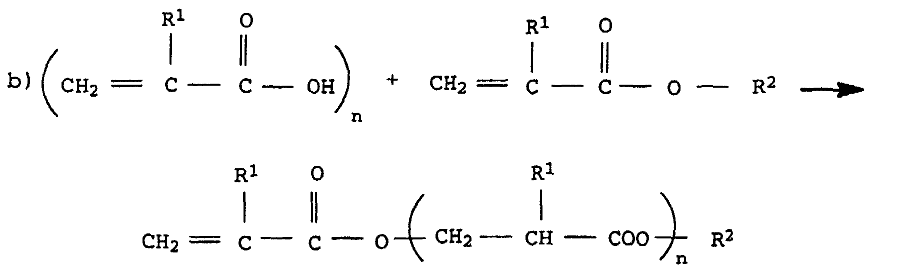 mit
- R¹=: Wasserstoff oder -CH₃,
- R²=: C₁-bis C₈-Alkyl und
- n =: eine ganze Zahl.

Insbesondere der beim erfindungsgemäßen Verfahren angewendete erhöhte Anteil an katalytisch wirksamer Säure begünstigt, zusammen mit einer erhöhten Reaktionstemperatur, offensichtlich die Rückspaltung der Oxiester in ihre Ausgangsverbindungen und gewährleistet so einen an Oxiestern besonders armen stationären Zustand in der Reaktionszone. In entsprechender Weise umfaßt der Austrag aus der Reaktionszone die Ausgangs- und Zielprodukt chemisch gebunden enthaltenden Oxiester nur im solch geringfügigem Ausmaß, daß eine Aufarbeitung des Austrags nicht mehr unabdingbar ist. Wenn nun aber dem Reaktionssystem via verminderte Oxiesterbildung nur noch in vermindertem Ausmaß Edukt und Zielprodukt entzogen wird, muß in notwendiger Weise die eduktbezogene Ausbeute ansteigen. Es überrascht, daß mit der erfindungsgemäß erzielten signifikanten Ausbeuteverbesserung nicht notwendigerweise eine signifikante Erhöhung der Dialkyletherbildung einhergeht.

### Beispiele (in allen Fällen wurde mit Phenothiazin stabilisiert)

Einem Umlaufverdampfer (2,5 1 Leervolumen, 2 1 Füllstand) als Reaktionszone wurden kontinuierlich 4 mol/h Acrylsäure, 4 mol/h an frischem n-Butanol und 2 mol/h n-Butanol aus der Butanolrückgewinnung kontinuierlich zugeführt (Verweilzeit= 2,3 h). Die Veresterungstemperatur wurde auf 126°C eingestellt. Als Säurekatalysator wurde Schwefelsäure eingesetzt. Der zugesetzte Gehalt an Schwefelsäure in der Reaktionszone, bezogen auf die Menge an darin enthaltenem Reaktionsgemisch, wurde auf einen stationären Wert von X Gew.-% eingestellt. Der Umlaufverdampfer war mit einer Glockenbodenkolonne (30 Böden, 50 mm Durchmesser) als aufgesetzter Rektifikationszone verbunden, über deren Kopf (Kopfdruck= 1 atm) gebildetes n-Butylacrylat als Bestandteil eines zusätzlich n-Butanol und Wasser enthaltenden Heteroazetropgemisches kontinuierlich abgezogen wurde. Das heteroazeotrope Destillat schied sich nach seiner Kondensation in eine organische und eine wäßrige Phase. Über Kopf der Glockenbodenkolonne wurden 350 ml/h der wäßrigen Phase und 180 ml/h der organischen Phase zurückgeführt. Die überschüssige organische Phase (620 ml/h) wurde kontinuierlich dem Mittelteil einer zweiten entsprechenden nachgeschalteten Glockenbodenkolonne zugeführt. In dieser n-Butanolrückgewinnungskolonne wurde das in der organischen Phase enthaltene n-Butanol mit darin ebenfalls enthaltenen Resten von Wasser sowie mit Teilen von n-Butylacrylat über Kopf abgetrennt (in kondensierter Form 278 ml/h, 83 Gew.-% davon n-Butanol) und auf direktem Weg in die Reaktionszone zurückgeführt. Der Kopf druck der zweiten Glockenbodenkolonne betrug 0,6 atm. Der Sumpf der n-Butanolrückgewinnungskolonne (Sumpftemperatur= 130°C) enthielt Roh-n-Butylacrylat in einer Reinheit von 99,5 Gew.-%. Nach einer nachgeschalteten rektifikativen Leichtsieder- und Schwersiederabtrennung wurde ein Rein-n-Butylacrylat mit einer Reinheit von ≥99,95 Gew.-% erhalten. Die Sumpfflüssigkeit der Schwersiederkolonne wurde in einer Menge von 30 g/h auf direktem Weg in die Reaktionszone zurückgeführt. Aus der Reaktionszone wurden während des Verfahrens kontinuierlich Y g/h an Reaktionsgemisch ausgetragen. Nachfolgende Tabelle zeigt die für verschiedene zugesetzt enthaltene stationäre Schwefelsäuregehalte X in der Reaktionszone erzielten Ausbeuten A an n-Butylacrylat, bezogen auf eingesetzte Acrylsäure (% der theoretisch erreichbaren Ausbeute). Die Angaben beziehen sich dabei auf die im Roh-n-Butylacrylat enthaltene Menge an n-Butyl-acrylat. Ferner weist die Tabelle die im Roh-n-Butylacrylate enthaltene Menge an Dialkylether aus (Gew.-%, bezogen auf im Roh-n-Butylacrylat enthaltenes n-Butylacrylat). Auch zeigt die Tabelle die erforderliche Menge an Austrag aus der Reaktionszone (einmal als Absolutmenge in g/h sowie einmal bezogen auf die im Roh-n-Butylacrylat enthaltenen Menge an n-Butylacrylat in Gew.-%).

Das Beispiel V3 bezieht sich auf eine Reaktionstemperatur von 100°C und das Beispiel V4 auf eine Reaktionstemperatur von 93°C. In entsprechender Weise betrug der Kopfdruck der der Reaktionszone aufgesetzten Rektifikationszone in diesen Beispielen 0,15 bzw. 0,135 atm.

**Tabelle**

| Bsp | X(Gew.-%) | A(% d.Th.) | Ether(Gew.-%) | Austrag | |
|---|---|---|---|---|---|
| | | | | [g/h] | bezgl. n-BA(Gew.-%) |
| V1 | 3,0 | 96,8 | 0,043 | 14,2 | 2,9 |
| V2 | 4,5 | 97,1 | 0,077 | 11,3 | 2,3 |
| B1 | 6,3 | 97,6 | 0,090 | 9,0 | 1,8 |
| B2 | 9,3 | 98,7 | 0,132 | 2,5 | 0,5 |
| B3 | 14,4 | 99,0 | 0,178 | 2,3 | 0,5 |
| B4 | 18,6 | 99,3 | 0,190 | 2,0 | 0,4 |
| V3 | 3,0 | 94,3 | 0,002 | 25,1 | 5,2 |
| V4 | 3,0 | 91,9 | 0,001 | 29,6 | 6,3 |

## Patentansprüche

1. Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase im Molverhältnis von 1(Alkanol): 1 ((Meth)acrylsäure)bis 2(Alkanol): 1 ((Meth)acrylsäure) bei erhöhter Temperatur und in Gegenwart von Schwefelsäure oder einer organischen Sulfonsäure als Katalysator, bei dem man die (Meth)acrylsäure, das Alkanol und den Säurekatalysator kontinuierlich einer Reaktionszone zuführt, nach einer Verweilzeit von einigen Stunden den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten, einen Kopfdruck von 0,1 bis 1 atm aufweisenden, Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und gegebenenfalls Wasser in die Reaktionszone zurückführt, aus der überschüssigen, den (Meth)acrylsäurealkylester enthaltenden organischen Phase den (Meth)acrylsäurealkylester in an sich bekannter Weise abtrennt und einen Teil des Reaktionsgemisches aus der Reaktionszone kontinuierlich austrägt, dadurch gekennzeichnet, daß
- die Reaktionstemperatur 100 bis 150°C beträgt,
- die Verweilzeit 1 bis 5 h beträgt und
- das in der Reaktionszone befindliche Reaktionsgemisch, bezogen auf die Menge an Reaktionsgemisch, als Säurekatalysator 5 bis 20 Gew.-% Schwefelsäure, oder eine zu einer solchen Schwefelsäuremenge äquimolare Menge einer organischen Sulfonsäure oder eine zu einer solchen Schwefelsäuremenge äquimolare Menge eines Gemisches aus Schwefelsäure und organischer Sulfonsäure zugesetzt enthält.

2. Verfahren nach Anspruch 1, dadurch gekennzeichnet, daß die im Reaktionsgemisch zugesetzt enthaltene Menge an Säurekatalysator, bezogen auf die Menge an Reaktionsgemisch, aus 5 bis 15 Gew.-% Schwefelsäure, einer zu einer solchen Schwefelsäuremenge äquimolaren Menge einer organischen Sulfonsäure oder einer zu einer solchen Schwefelsäuremenge äquimolaren Menge eines Gemisches aus Schwefelsäure und organischer Sulfonsäure besteht.

3. Verfahren nach Anspruch 1, dadurch gekennzeichnet, daß die im Reaktionsgemisch zugesetzt enthaltene Menge an Säurekatalysator, bezogen auf die Menge an Reaktionsgemisch, aus 5 bis 10 Gew.-% Schwefelsäure, einer zu einer solchen Schwefelsäuremenge äquimolaren Menge einer organischen Sulfonsäure oder einer zu einer solchen Schwefelsäuremenge äquimolaren Menge eines Gemisches aus Schwefelsäure und organischer Sulfonsäure besteht.

4. Verfahren nach Anspruch 1 bis 3, dadurch gekennzeichnet, daß die Untergrenze des Mengenbereichs des zugesetzt enthaltenen Säurekatalysators 5,5 Gew.-% beträgt.

5. Verfahren nach Anspruch 1 bis 3, dadurch gekennzeichnet, daß die Untergrenze des Mengenbereichs des zugesetzt enthaltenen Säurekatalysators 6 Gew.-% beträgt.

6. Verfahren nach Anspruch 1 bis 3, dadurch gekennzeichnet, daß die Untergrenze des Mengenbereichs des zugesetzt enthaltenen Säurekatalysators >8 Gew.-% beträgt.

7. Verfahren nach Anspruch 1 bis 6, dadurch gekennzeichnet, daß der zugesetzt enthaltene Säurekatalysator Methansulfonsäure, Benzolsulfonsäure, Dodecylbenzolsulfonsäure und/oder p-Toluolsulfonsäure umfaßt.

8. Verfahren nach Anspruch 1 bis 7, dadurch gekennzeichnet, daß die Reaktionstemperatur >100 bis 140°C beträgt.

9. Verfahren nach Anspruch 1 bis 8, dadurch gekennzeichnet, daß die Reaktionstemperatur 120 bis 130°C beträgt.

10. Verfahren nach Anspruch 1 bis 9, dadurch gekennzeichnet, daß die Verweilzeit in der Reaktionszone 2 bis < 4 h beträgt.

11. Verfahren nach Anspruch 1 bis 10, dadurch gekennzeichnet, daß die Verweilzeit in der Reaktionszone 3 bis < 4 h beträgt.

12. Verfahren nach Anspruch 1 bis 11, dadurch gekennzeichnet, daß man der Reaktionszone das Ausgangsalkanol und die Ausgangssäure im molaren Verhältnis 1,3 bis 1,7 : 1 zuführt.

13. Verfahren nach Anspruch 1 bis 12, dadurch gekennzeichnet, daß der Kopf druck der der Reaktionszone aufgesetzten Rektifikationszone 1 atm beträgt.

14. Verfahren nach Anspruch 1 bis 13, dadurch gekennzeichnet, daß die zu veresternde Säure Acrylsäure ist.

15. Verfahren nach Anspruch 1 bis 14, dadurch gekennzeichnet, daß das zu veresternde Alkanol ein C₁- bis C₄-Alkanol ist.

16. Verfahren nach Anspruch 1 bis 15, dadurch gekennzeichnet, daß das zu veresternde Alkanol n-Butanol ist.

## Claims

1. A process for the continuous preparation of alkyl esters of (meth)acrylic acid by reacting (meth)acrylic acid and a monohydric alkanol of 1 to 8 carbon atoms in a homongeneous, liquid, solvent-free phase in a molar ratio of from 1 (alkanol) : 1 ((meth)acrylic acid) to 2 (alkanol) : 1 ((meth)acrylic acid) at elevated temperatures and in the presence of sulfuric acid or of an organic sulfonic acid as catalyst, in which the (meth)acrylic acid, the alkanol and the acid catalyst are fed continuously to a reaction zone, after a residence time of a few hours the resulting alkyl (meth)acrylate is separated off by rectification, as part of at least one azeotropic mixture consisting of water or water and starting alkanol as further components in addition to the alkyl (meth)acrylate, via the top of a rectification zone attached to the reaction zone and having a top pressure of from 0.1 to 1 atm, the distillate obtained is separated into at least one organic phase containing the alkyl (meth)acrylate and into at least one aqueous phase containing water, some of said organ; ic phase is recycled to the rectification zone via the top and, if required, water is recycled to the reaction zone, the alkyl (meth)acrylate is separated off in a manner known per se from the excess organic phase containing the alkyl (meth)acrylate and some of the reaction mixture is discharged continuously from the reaction zone, wherein
- the reaction temperature is from 100 to 150°C,
- the residence time is from 1 to 5 hours and
- the reaction mixture present in the reaction zone contains, as an added acid catalyst, from 5 to 20 % by weight, based on the amount of reaction mixture, of sulfuric acid or an equimolar amount, based on such an amount of sulfuric acid, of an organic sulfonic acid or an equimolar amount, based on such an amount of sulfuric acid, of a mixture of sulfuric acid and organic sulfonic acid.

2. A process as claimed in claim 1, wherein the amount of acid catalyst added to the reaction mixture consists of from 5 to 15 % by weight, based on the amount of reaction mixture, of sulfuric acid, of an equimolar amount, based on such an amount of sulfuric acid, of an organic sulfonic acid or of an equimolar amount, based on such an amount of sulfuric acid, of a mixture of sulfuric acid and organic sulfonic acid.

3. A process as claimed in claim 1, wherein the amount of acid catalyst added to the reaction mixture consists of from 5 to 10 % by weight, based on the amount of reaction mixture, of sulfuric acid, of an equimolar amount, based on such an amount of sulfuric acid, of an organic sulfonic acid or of an equimolar amount, based on such an amount of sulfuric acid, of a mixture of sulfuric acid and organic sulfonic acid.

4. A process as claimed in any of claims 1 to 3, wherein the lower limit of the range of the amounts of added acid catalyst is 5.5 % by weight.

5. A process as claimed in any of claims 1 to 3, wherein the lower limit of the range of the amounts of added acid catalyst is 6 % by weight.

6. A process as claimed in any of claims 1 to 3, wherein the lower limit of the range of the amounts of added acid catalyst is >8 % by weight.

7. A process as claimed in any of claims 1 to 6, wherein the added acid catalyst comprises methanesulfonic acid, benzenesulfonic acid, dodecylbenzenesulfonic acid or p-toluenesulfonic acid.

8. A process as claimed in any of claims 1 to 7, wherein the reaction temperature is from >100 to 140°C.

9. A process as claimed in any of claims 1 to 8, wherein the reaction temperature is from 120 to 130°C.

10. A process as claimed in any of claims 1 to 9, wherein the residence time in the reaction zone is from 2 to < 4 hours.

11. A process as claimed in any of claims 1 to 10, wherein the residence time in the reaction zone is from 3 to < 4 hours.

12. A process as claimed in any of claims 1 to 11, wherein the starting alkanol and the starting acid are fed to the reaction zone in a molar ratio of from 1.3:1 to 1.7:1.

13. A process as claimed in any of claims 1 to 12, wherein the top pressure of the rectification zone attached to the reaction zone is 1 atm.

14. A process as claimed in any of claims 1 to 13, wherein the acid to be esterified is acrylic acid.

15. A process as claimed in any of claims 1 to 14, wherein the alkanol to be esterified is a C₁-C₄-alkanol.

16. A process as claimed in any of claims 1 to 15, wherein the alkanol to be esterified is n-butanol.

## Revendications

1. Procédé de préparation continue d'esters alkyliques de l'acide (méth)acrylique par la réaction de l'acide (méth)acrylique et d'alcanols monohydroxylés comportant de 1 à 8 atomes de carbone en phase homogène, liquide, dépourvue de solvant, dans le rapport molaire de 1(alcanol):1[acide (méth)acrylique]bis 2(alcanol):1[acide (méth)acrylique], à température élevée et en présence d'acide sulfurique ou d'un acide sulfonique organique, à titre de catalyseur, conformément auquel on introduit l'acide (méth)acrylique, l'alcanol et le catalyseur acide en continu dans une zone de réaction, après une durée de séjour de quelques heures, on sépare par rectification le (méth)acrylate d'alkyle formé à titre de constituant, au moins un azéotrope constitué, outre du (méth)acrylate d'alkyle, d'eau ou d'eau et d'alcanol de départ à titre de constituants supplémentaires, en tête d'une colonne de rectification montée sur la zone de réaction, présentant une pression de tête de 0,1 à 1 atm, le distillat présent, en au moins une phase organique contenant le (méth)acrylate d'alkyle et en au moins une phase aqueuse contenant de l'eau, on renvoie une partie de la phase organique contenant le (méth)acrylate d'alkyle en tête de la zone de rectification et on renvoie éventuellement l'eau dans la zone de réaction, on sépare le (méth)acrylate d'alkyle de la phase organique contenant le (méth)acrylate d'alkyle, excédentaire, d'une manière en soi connue et on évacue en continu une partie du mélange réactionnel de la zone de réaction, caractérisé en ce que
- la température réactionnelle varie de 100 à 150°C,
- la durée de séjour varie de 1 à 5 heures et
- le mélange réactionnel qui se trouve dans la zone de réaction contient, par rapport à la quantité de mélange réactionnel, à titre de catalyseur acide, 5 à 20% en poids d'acide sulfurique, ou une quantité équimolaire à une telle proportion d'acide sulfurique, d'un acide sulfonique organique, ou une quantité équimolaire correspondant à une telle proportion d'acide sulfurique, d'un mélange constitué d'acide sulfurique et d'acide sulfonique organique, ajouté.

2. Procédé suivant la revendication 1, caractérisé en ce que la quantité ajoutée contenue dans le mélange réactionnel en catalyseur acide se compose, par rapport à la quantité de mélange réactionnel, de 5 à 15% en poids d'acide sulfurique, d'une quantité équimolaire à une telle proportion d'acide sulfurique, d'un acide sulfonique organique, ou d'une quantité équimolaire à une telle proportion d'acide sulfurique, d'un mélange constitué d'acide sulfurique et d'acide sulfonique organique.

3. Procédé suivant la revendication 1, caractérisé en ce que la quantité ajoutée contenue dans le mélange réactionnel de catalyseur acide se compose, par rapport à la quantité de mélange réactionnel, de 5 à 10% en poids d'acide sulfurique, d'une quantité équimolaire à une telle proportion d'acide sulfurique, d'un acide sulfonique organique, ou d'une quantité équimolaire à une telle proportion d'acide sulfurique, d'un mélange d'acide sulfurique et d'acide sulfonique organique.

4. Procédé suivant l'une quelconque des revendications 1 à 3, caractérisé en ce que la limite inférieure de la plage des proportions du catalyseur acide ajouté contenu est de 5,5% en poids.

5. Procédé suivant l'une quelconque des revendications 1 à 3, caractérisé en ce que la limite inférieure de la plage des proportions du catalyseur acide ajouté contenu est de 6% en poids.

6. Procédé suivant l'une quelconque des revendications 1 à 3, caractérisé en ce que la limite inférieure de la plage des proportions du catalyseur acide ajouté contenu est >8% en poids.

7. Procédé suivant l'une quelconque des revendications 1 à 6, caractérisé en ce que le catalyseur acide ajouté contenu comprend de l'acide méthanesulfonique, de l'acide benzènesulfonique, de l'acide dodécylbenzènesulfonique et/ou de l'acide p-toluènesulfonique.

8. Procédé suivant l'une quelconque des revendication 1 à 7, caractérisé en ce que la température réactionnelle varie >100 à 140°C.

9. Procédé suivant l'une quelconque des revendications 1 à 8, caractérisé en ce que la température réactionnelle varie de 120 à 130°C.

10. Procédé suivant l'une quelconque des revendications 1 à 9, caractérisé en ce que la durée de séjour dans la zone de réaction varie de 2 à < 4 heures.

11. Procédé suivant l'une quelconque des revendications 1 à 10, caractérisé en ce que la durée de séjour dans la zone de réaction varie de 3 à < 4 heures.

12. Procédé suivant l'une quelconque des revendications 1 à 11, caractérisé en ce que l'on introduit l'alcanol de départ et l'acide de départ dans le rapport molaire de 1,3 à 1,7:1 dans la zone de réaction.

13. Procédé suivant l'une quelconque des revendications 1 à 12, caractérisé en ce que la pression de tête de la zone de rectification surmontant la zone de réaction est de 1 atm.

14. Procédé suivant l'une quelconque des revendications 1 à 13, caractérisé en ce que l'acide à estérifier est l'acide acrylique.

15. Procédé suivant l'une quelconque des revendications 1 à 14, caractérisé en ce que l'alcanol à estérifier est un alcanol en C₁ à C₄.

16. Procédé suivant l'une quelconque des revendications 1 à 15, caractérisé en ce que l'alcanol à estérifier est le n-butanol.
